# EUROPEAN PATENT SPECIFICATION

(11) **EP 0 901 379 B1**
(45) Date of publication and mention of the grant of the patent: **06.09.2000**
(21) Application number: 97926647.5
(22) Date of filing: 21.05.1997
(51) Int. Cl.: A61K 38/16

(54) **TYPE-2 CHEMOKINE BINDING PROTEINS AND METHODS OF USE THEREFOR**
TYP-2 CHEMOKINE-BINDENDE PROTEINE UND VERFAHREN ZU DEREN VERWENDUNG
PROTEINES DE FIXATION DE LA CHIMIOKINE DE TYPE 2 ET LEURS PROCEDES D'UTILISATION

(30) Priority: 22.05.1996 US 18137 P
(43) Date of publication of application: 17.03.1999
(60) Divisional application: 00103719.1
(73) Proprietor: UNIVERSITY OF ALBERTA, Edmonton, Alberta T6G 2E1 (CA)
(72) Inventor: McFADDEN, D. Grant, Edmonton, Alberta T6H 0H6 (CA); LUCAS, Alexandra, Edmonton, Alberta T6H 0H6 (CA)
(74) Representative: Vossius, Volker, Dr.
(86) International application number: US9708590
(87) International publication number: WO9744054

(56) References cited:
- WO-A-92/17583
- WO-A-96/33730
- WO-A-97/11714
- SCHREIBER M. ER AL.: "The Myxoma Virus TNF-Receptor Homologue (T2) Inhibits Tumor Necrosis Factor-alpha in a Species-Specific Fashion" VIROLOGY, vol. 204, no. 2, 1 November 1994, pages 692-705, XP002057409 cited in the application
- GRAHAM K A ET AL: "The T1 -35kDa family of poxvirus -secreted proteins bind chemokines and modulate leukocyte influx into virus-infected tissues." VIROLOGY 229 (1). 1997. 12-24, XP002057410

## Description

### BACKGROUND OF THE INVENTION

### 1. Field of the Invention

This invention relates generally to the field of immunology and specifically to a chemokine binding protein encoded by a variety of poxviruses and methods of use therefor.

### 2. Description of Related Art

It is becoming increasingly clear that viruses which make their living within cells of higher-order vertebrates must have evolved to specifically avoid the host immune system (Gooding, L., *Cell,* 91:5-7, 1992; Marrack, P. and Kappler, J., *Cell,* 76:323-332, 1994; Smith, G., *Trends in Micro.,* 82:80-88, 1994). In fact, virus survival is dependent upon strategies which can evade, suppress, counteract, or otherwise confound the myriad of host responses to a foreign invader. The selection pressure conferred by the effector arms of the immune system can clearly be a powerful element of evolutionary pressure, and all eukaryotic viruses existing today contain imprints or remnants of their battles with the immune system, either as encoded proteins or as evidenced by their particular biological survival strategies.

The larger DNA viruses (i.e. the adenoviruses, herpesviruses, iridoviruses and poxviruses) specifically encode proteins that function to protect the virus from immune recognition and/or clearance by the infected host. Such "subversive" viral proteins are now providing information concerning the functional operations of the immune system, and it is likely that many more discoveries of new members of this growing family will be identified in the future.

In the 1980's the term "virokine" was proposed to describe virus-encoded proteins secreted from infected cells which function by mimicking extracellular signaling molecules such as cytokines or other secreted regulators important for the host immune repertoire (Kotwal, G. and Moss, B., *Nature,* 335: 176-178, 1988). Later, in the 1990's the term "viroceptor" was introduced to account for the observation that some virus encoded proteins that mimic important cellular receptors and function by diverting host cytokines away from their normal receptors, thus interrupting the immune circuitry at its earliest stages (Upton, *et al., Virology,* 184:370, 1991; Schreiber and McFadden, *Virology,* 204:692-705, 1994).

Recent studies on a particular poxvirus, myxoma virus, have shown that the virus disrupts the immune system by a variety of strategies (McFadden and Graham, *Seminars in Virology,* 5:421-429, 1994). Myxoma virus is the infectious agent of a virulent systemic disease of domestic rabbits called myxomatosis. Originally described in the last century, myxoma was the first virus pathogen discovered for a laboratory animal and was the first viral agent ever deliberately introduced into the environment for the explicit purpose of pest eradication. Since its release into the Australian and European feral rabbit populations more than 40 years ago, the field strains of both the rabbit and virus have been subjected to mutual evolutionary and selective pressures that have resulted in a steady-state enzootic in the inoculated areas (Fenner, F. and Ratcliffe, F.N., "Myxomatosis", *Cambridge University Press, London,* 1965).

Myxoma shares many of the biologic features associated with other poxviruses, namely cytoplasmic location of replication and a large double stranded DNA genome (160 kilobases). Multiple lines of evidence indicate that myxoma, like all poxviruses, encodes multiple gene products whose function is to permit the spread and propagation of the virus in a variety of host tissues. Some of these viral proteins specifically counteract or subvert the development of the host inflammatory response and acquired cellular immunity, and poxviruses in general have been a rich source of such immunomodulatory proteins (Turner, P.C., and Moyer, R.W., *Cur. Top. Microbiol. Imm.,* 163:125-152, 1990; Buller, R.M.L., and Palumbo, G.J., *Micro. Dev.,* 55:80-122, 1991; Smith, G.L., *J., Gen. Virol.,* 94:1725-1740, 1993; McFadden, G., (Ed.), "Viroceptors, virokines and related immune modulators encoded by DNA viruses", *R.G. Landes Co.,* Austin Texas, 1995).

Examples of such immunomodulatory gene products include myxoma growth factor (MGF), which stimulates neighboring cells in a paracrine-like fashion via the cellular epidermal growth factor receptor (Upton, *et al., J. Virol.,* 61:1271-1275, 1987; Opgenorth, *et al., Virol.,* 186:185-191, 1992; Opgenorth, *et al., Virol.,* 192:701-708, 1992; Opgenorth, *et al., J. Virol.,* 66:4720-4731, 1992); Serp 1, a secreted glycoprotein with serine protease inhibitor activity, that prevents development of the early inflammatory response (Upton, *et al., Virol.,* 179:628-631,1990; Lomas, *et al., JBC,* 268:516-521, 1993; Macen, *et al., Virol.,* 195:348-363, 1993); T2, a secreted viral homologue of the cellular tumor necrosis factor (TNF) receptor superfamily, that binds and inhibits rabbit TNF (Smith, *et al., BBRC,* 176:335-342, 1991; Schreiber, M. and McFadden, G., *supra,* 1994; Upton, *et al., supra,* 1991); T7, a secreted viral homologue of the cellular interferon-γ receptor, that binds and inhibits rabbit interferon-γ (Upton, *et al., Science,* 258:1369, 1992; Upton and McFadden, *Methods in Molecular Genetics,* 4:383, 1994; Mossman, *et al.,* In: "Viroceptors, virokines and related immune modulators" p. 41-54 Ed. McFadden, *R.G. Landers, Co.,* 1995); and M11L, a surface receptor-like protein that interferes within the inflammatory response by an unknown mechanism (Opgenorth, *et al., supra;* Graham, *et al., Virol,* 191:112-124, 1992);

Immunomodulatory proteins also include chemotactic cytokines, called "chemokines". Chemokines are small molecular weight immune ligands which are chemoattractants for leukocytes, such as especially neutrophils, basophils, monocytes and T cells. There are two major classes of chemokines which both contain four conserved cysteine residues which form disulfide bonds in the tertiary structure of the proteins. The α class is designated C-X-C (where X is any amino acid), which includes IL-8, CTAP-III, gro/MGSA and ENA-78; and the β class, designated C-C, which includes MCP-1, MIP-1α and β, and regulated on activation, normal T expressed and secreted protein (RANTES). The designations of the classes are according to whether an intervening residue spaces the first two cysteines in the motif. In general, most C-X-C chemokines are chemoattractants for neutrophils but not monocytes, whereas C-C chemokines appear to attract monocytes but not neutrophils. Recently, a third group of chemokines, the "C" group, was designated by the discovery of a new protein called lymphotactin (Kelner, *et al., Science,* 266:1395-1933, 1994). The chemokine family is believed to be critically important in the infiltration of lymphocytes and monocytes into sites of inflammation.

It is highly likely that more immunomodulatory viral genes remain to be discovered. Not only will these and related gene products provide useful tools to dissect out the different arms of the host antiviral defense mechanisms, but they may also provide new probes to identify novel elements of the cellular immune repertoire and new classes of drugs to suppress inflammation and dysregulation of the immune system.

### SUMMARY OF THE INVENTION

The present invention describes a new family of soluble virus-specific inhibitors for a class of cytokines which are involved in leukocyte chemotaxis and are collectively referred to as "chemokines". These proteins are designated type 2 chemokine binding proteins (type-2 CBP) and are a family of poxviruses proteins related to the T1 protein encoded by Shope fibroma virus and myxoma virus (SFV-T1). The type-2 CBP and related functionally homologues are useful for treatment of a variety of inflammatory disorders in which excessive influx of leukocytes is associated with the pathogenic process.

### BRIEF DESCRIPTION OF THE DRAWINGS

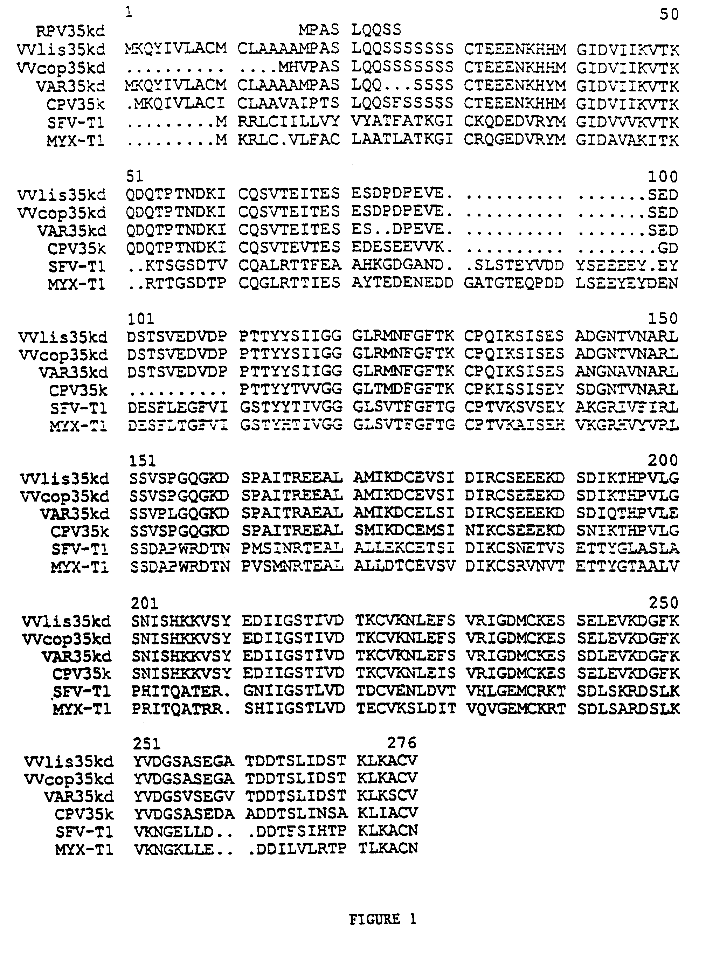

FIGURE 1 shows the sequence alignment of the known members of the type-2 CBP family of the poxvirus proteins that bind to chemotactic cytokines (chemokines). The RPV 35KDa sequence is incomplete.

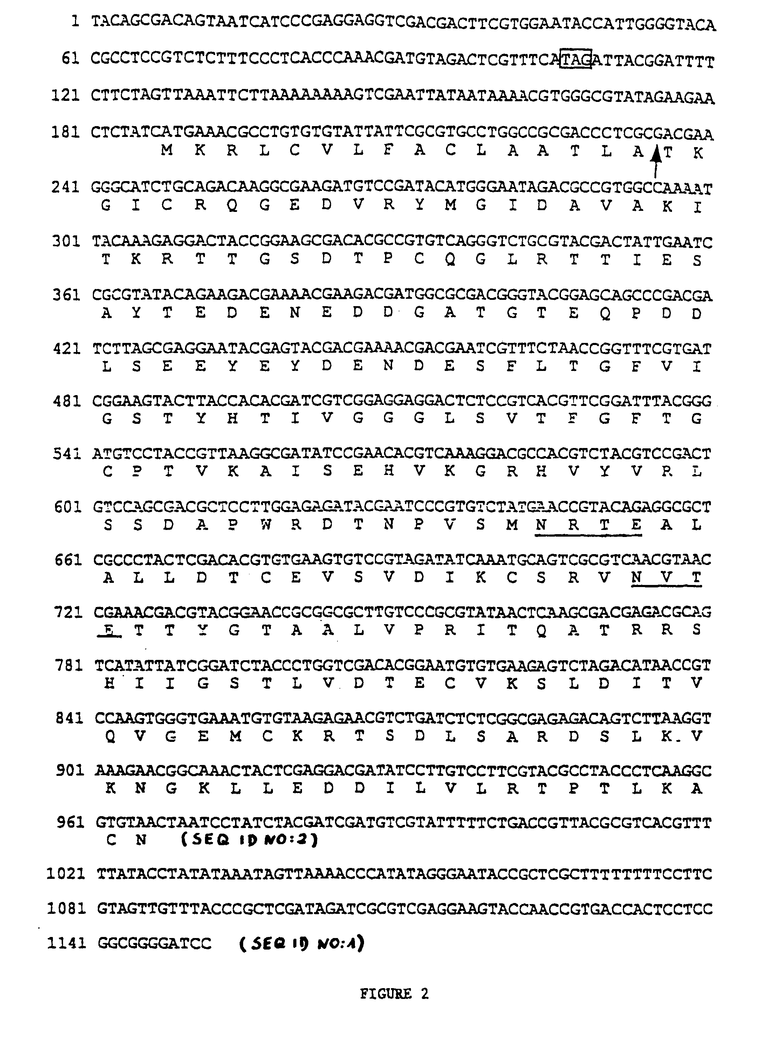

FIGURE 2 shows the nucleotide sequence of the Tl gene of myxoma, which expresses a type-2 CBP. The boxed nucleotide triplet is the stop codon for the adjacent T2 gene (TNF-receptor homolog), the arrow denotes the predicted signal peptide cleavage site, and the underlined amino acids are the two predicted N-glycosylation sites for the T1 protein (SEQ ID NO:1 and SEQ ID NO:2).

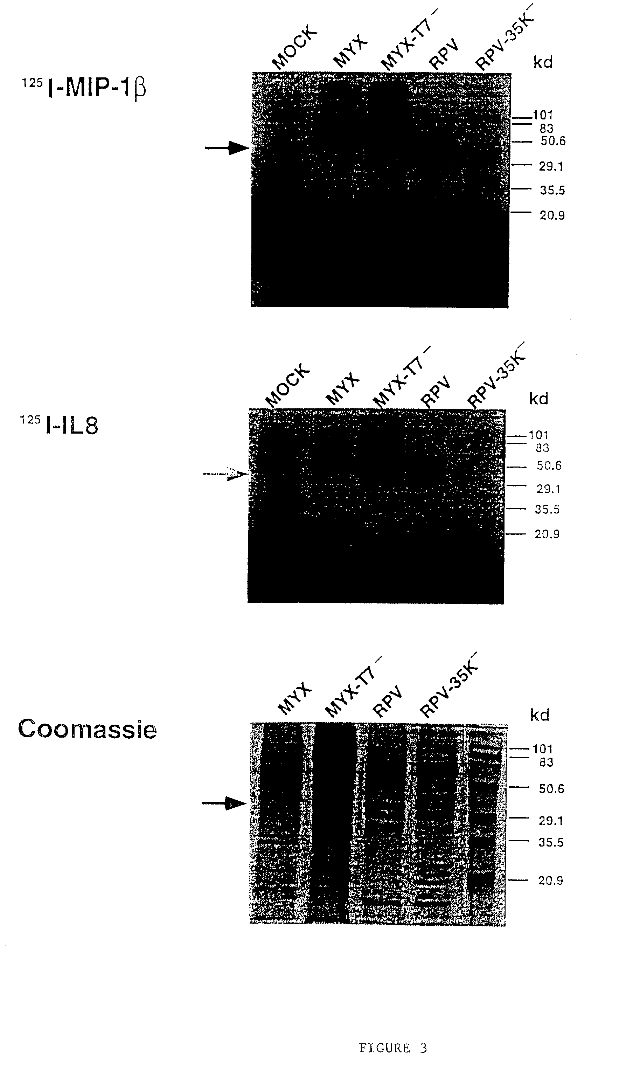

FIGURE 3 shows that the 35KDa protein of rabbit poxvirus (RPV), one of the members of the type-2 CBP family, binds members of the C-C family of chemokines (MIP-1 beta, upper panel), and the C-X-C family (11-8, middle panel). In the upper two panels, the radiolabelled ligands are cross-linked to viral proteins secreted from control infected BGMK cells (MOCK), or cells infected with myxoma (MYX), a T7-deletion mutant of myxoma (MYX-T7-), rabbit poxvirus (RPV), and a 35KDa-deletion mutant of RPV (RPV-35K-). The cross-linked complexes between the ligand and the viral proteins are indicated with the arrows. Although the myxoma type-1 CBP protein (T7) also binds chemokines, the type-2 CBP protein (T1) is virtually the same size (ca 35KDa), as shown in the bottom panel. Both T1 (type 2) and T7 (type 1) proteins of myxoma thus bind chemokines, but only the 35KDa protein of RPV (type 2) has this activity.

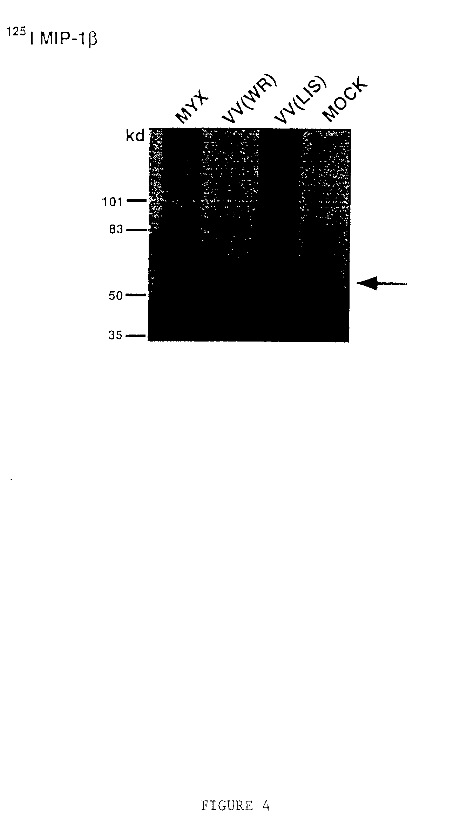

FIGURE 4 shows that the 35KDa secreted protein from vaccinia (strain Lister), but which is missing from strain WR, binds the chemokine MIP-1β similarly to the 35KDa secreted protein of RPV. The arrow marks the complex between MIP-1B and the 35KDa protein secreted from BGMK cells infected with vaccinia strain Lister, but not strain WR.

### DETAILED DESCRIPTION OF THE INVENTION

The findings of the present invention provide an important new source of anti-immune proteins which have the potential to treat a wide range of immunopathological conditions associated with the trafficking of lymphocytes and monocytes from the circulation to tissue sites during inflammation and immune responses to damage, infection and various disease states.

The cloned and sequenced type-2 CBP genes are not secreted homologues of the known chemokine receptors, which all possess seven membrane-spanning domains (called "serpentines") as described in recent reviews (Kelvin, D.J., *et al., J. Leukocyte Biol.,* 54:604-612, 1993; Murphy, P.M., *Ann. Rev. Imm.,* 12:593-633, 1994; Horuk, R., *Imm. Today.,* 15:169-174, 1994; and Horuk, R., *Trends in Pharm. Sci.,* 15:159-165, 1994). Although some DNA viruses do encode homologues of such serpentine receptors (Ahuja, S.K., *et al., Imm. Today,* 15:281-287, 1994), including at least one gene candidate in a poxvirus (Massung, R.F., *et al., Virology,* 197:511-528, 1994), the type-2 CBP of the present invention is not a member of this particular receptor family.

The exemplary type-2 chemokine binding protein (type-2 CBP) of the invention is one of the secreted proteins from cells infected with Shope fibroma virus and is encoded by the T1 open reading frame (Upton, *et al., Virology,* 160:20-30, 1987; and GenBank Accession No: P25946). This protein has significant sequence similarity to the secreted 35kDa proteins of vaccinia (strains Copenhagen and Lister) and rabbit poxvirus. Further, the type-2 CBP proteins are distinct from the myxoma M-T7 protein which specifically binds rabbit IFN-γ, but not mouse or human IFN-γ (Mossman, *et al., J. Biol. Chem.,* 270:3031-3038, 1995) but which also binds chemokines and is designated as a type-1 chemokine binding protein (previously denoted chemokine binding protein-1 (CBP-1)).

The term "chemokine binding protein" refers to a protein which binds to and inhibits one or more chemokines. A "chemokine" is a class of cytokines which are responsible for leukocyte chemotaxis. The α class of chemokines is designated C-X-C (where X is any amino acid), which includes interleukin-8 (I1-8), connective tissue activating protein III (CTAP-III), melanocyte growth stimulatory activity (MGSA) gro/MGSA, IFN-γ inducible protein (IP-10), neutrophil activating peptide 2 (NAP2), β-thromboglobulin and epithelial-derived neutrophil attractant-78 (ENA-78); and the β class, designated C-C, which includes T-cell activation gene-3 (TCA-3), monocyte chemotactic proteins (MCP-1, 2, and 3), macrophage inflammatory proteins (MIP-1α and β), and regulated on activation, normal T expressed and secreted protein (RANTES).

Other chemokines can be detected by methods commonly used in the art. For example, a molecule may be tested using the Boyden chamber, which is the preferred microchemotaxis assay sytem for *in vitro* investigation of chemo-attractant substances. A series of wells is formed into a plexiglass block, each well consisting of two chambers, upper and lower, which are separated by any one of several types of porous filters, such as nitrocellulose and polycarbonate, for example. The cell of interest, for example peripheral blood mononuclear cells (PBMC) are added to the top chamber of each well and the test substance, *e.g.*, the chemoattractant, is added to the bottom chamber. If the cells in the top chamber are attracted to the substance in the bottom chamber, they will migrate along the theoretical concentration gradient which exists in solution and crawl through the pores of the filter and adhere to the bottom side of that filter.

Polypeptides suspected of being members of the chemokine family can now be screened using the CBP of the invention. Therefore, in one embodiment, the invention provides a method for screening and identifying novel chemokines comprising contacting free or matrix-bound CBP of the invention with a composition suspected of containing one or more chemokines and detecting binding of the CBP to the composition.

If desirable, various labels can be used as means for detecting binding of CBP to a chemokine. Chemokines or the CBP can be directly or indirectly detectably labeled, for example, with a radioiscope, a fluorescent compound, a bioluminescent compound, a chemiluminescent compound, a metal chelator or an enzyme. Those of ordinary skill in the art will know of other suitable labels or will be able to ascertain such, using routine experimentation.

In one embodiment, the invention provides a method for treating an immunopathological disorder in a subject comprising administering to the subject a therapeutically effective amount of an anti-inflammatory protein characterized as having a molecular weight of approximately 30-40 kD, depending on the extent of glycosylations as determined by reduced SDS-PAGE, having amino acid sequence homology with the SFV T1 or RPV 35kDa homolog and having the property of being secreted from infected cells. The term "anti-inflammatory" refers to reduction or suppression of an inflammatory response.

The glycosylated and secreted form of the type-2 CBP of the invention has an apparent molecular weight of approximately 35-40 kD as determined under reducing conditions on an SDS-PAGE. In addition, the protein has homology with the SFV Tl and RPV 35kDa secreted proteins. The term "homology" refers to the extent of identity between the type-2 CBP and other family members at the amino acid level. Preferably, the type-2 CBP has between 50-95% amino acid sequence homology with the SFV T1 protein. The homology requirement is not stringent, however, the type-2 CBP must retain the biological function of interacting with human chemokines. In other words, the homology is sufficient as long as the type-2 CBP binds and inhibits chemokines.

The invention includes a functional polypeptide, type-2 CBP, and functional fragments thereof. As used herein, the term "functional polypeptide" refers to a polypeptide which possesses a biological function or activity which is identified through a defined functional assay and which is associated with a particular biologic, morphologic, or phenotypic response. Functional fragments of the type-2 CBP polypeptide, include fragments of type-2 CBP as long as the activity of type-2 CBP remains (e.g., binding to chemokines). Smaller peptides containing the biological activity of type-2 CBP are included in the invention. Such peptides can be assayed for binding to chemokines by methods commonly known to those of skill in the art, including methods described in the EXAMPLES herein. The biological function can vary from a polypeptide fragment as well as an epitope to which an antibody molecule can bind to a large polypeptide which is capable of participating in the characteristic induction or programming of phenotypic changes within a cell. A "functional polynucleotide" denotes a polynucleotide which encodes a functional polypeptide as described herein.

Minor modifications of the type-2 CBP primary amino acid sequence may result in proteins which have substantially equivalent activity as compared to the type-2 CBP polypeptide described herein. Such modifications may be deliberate, as by site-directed mutagenesis, or may be spontaneous. All of the polypeptides produced by these modifications are included herein as long as the activity of type-2 CBP is retained. Further, deletion of one or more amino acids can also result in a modification of the structure of the resultant molecule without significantly altering its activity. This can lead to the development of a smaller active molecule which would have broader utility. For example, it is possible to remove amino or carboxy terminal amino acids which may not be required for type-2 CBP activity.

The type-2 CBP polypeptide of the invention also includes conservative variations of the polypeptide sequence. The term "conservative variation" as used herein denotes the replacement of an amino acid residue by another, biologically similar residue.

Examples of conservative variations include the substitution of one hydrophobic residue such as isoleucine, valine, leucine or methionine for another, or the substitution of one polar residue for another, such as the substitution of arginine for lysine, glutamic for aspartic acids, or glutamine for asparagine, and the like. The term "conservative variation" also includes the use of a substituted amino acid in place of an unsubstituted parent amino acid provided that antibodies raised to the substituted polypeptide also immunoreact with the unsubstituted polypeptide.

Examples of viral sources of the type-2 CBP used in the method of the present invention include myxoma virus, vaccinia (strains Lister and Copenhagen), Shope fibroma virus, rabbitpox and other mammalian pox viruses, as long as the type-2 CBP has the biological function of an anti-inflammatory protein characterized as having a molecular weight of approximately 30-40 kD, depending on extent of glycosylation having homology with SFV TI protein homolog, and having the biological function of this family of proteins.

An immunopathological disorder treated by the method of the invention may be associated with production of chemokines and resultant accumulation of reactive leukocytes at afflicted tissues. The method comprises administering to the subject a therapeutically effective amount of type-2 CBP. The term "immunopathological disorder" refers to any disease which involves the immune response or immunity in general. "Therapeutically effective" as used herein, refers to that amount of type-2 CBP that is of sufficient quantity to ameliorate the cause of the immunopathological disorder. "Ameliorate" refers to a lessening of the detrimental effect of the disorder in the patient receiving the therapy. The subject of the invention is preferably a human, however, it can be envisioned that any animal with an immunopathological disorder can be treated by the method of the invention, for example, a SCID mouse grafted with human bone marrow (humanized SCID). Examples of immunopathological disorders which can be treated by the method of the invention include acquired immunodeficiency disorder (AIDS), toxic shock syndrome, allograft rejection, artherosclerotic plaque growth, ultraviolet and radiation responses, and disorders associated with the activation of T cells, B cells, macrophages, and other inflammatory leukocytes during the immune response and the acute phase response and disorders associated with advanced cancer such as tumor necrosis factor-mediated cachexia.

The invention provides a method of treating or ameliorating an immunopathological disorder including endotoxemia or septic shock (sepsis), or one or more of the symptoms of sepsis comprising administering to a subject displaying symptoms of sepsis or at risk for developing sepsis, a therapeutically effective amount of type-2 CBP. The term "ameliorate" refers to a decrease or lessening of the symptoms of the disorder being treated.

A patient who exhibits the symptoms of an immunopathological disorder may be treated with an antibiotic or antiviral agent in addition to the treatment with type-2 CBP. Typical antibiotics include an aminoglycoside, such as gentamycin or a betalactam such as penicillin, or cephalosporin. Therefore, a therapeutic method of the invention includes administering a therapeutically effective amount of type-2 CBP substantially simultaneously with administration of a bactericidal amount of an antibiotic or sufficient amount of an anti-viral compound.

The term "bactericidal amount" as used herein refers to an amount sufficient to achieve a bacteria-killing blood concentration in the patient receiving the treatment. The bactericidal amount of antibiotic generally recognized as safe for administration to a human is well known in the art, and as is known in the art, varies with the specific antibiotic and the type of bacterial infection being treated. Preferably, administration of type-2 CBP occurs within about 48 hours and preferably within about 2-8 hours, and most preferably, substantially concurrently with administration of the antibiotic.

Administration of a type-2 CBP in the method of the invention may also be used for ameliorating post-reperfusion injury. When treating arterial thrombosis, induction of reperfusion by clot lysing agents such as tissue plasminogen activator (t-PA) is often associated with tissue damage. Such tissue damage is thought to be mediated at least in part by leukocytes including but not limited to polymorphonuclear leukocytes (PMN). Therefore administration of the type-2 CBP would block leukocyte or PMN-endothelial interactions, and thereby diminish or prevent post-reperfusion injury. Administration of type-2 CBP is also useful for prevention of new onset and recurrent atherosclerotic plaque growth after arterial injury. Restenosis and new growth of plaque is believed to be exacerbated by the local inflammatory response to the internal layer of the artery wall.

The method of the invention is also useful for treatment of inflammation due to allergic or autoimmune disorders. Examples of allergic disorders include allergic rhinitis, asthma, atopic dermatitis, and food allergies. Examples of autoimmune disorders, where the immune system attacks the host's own tissues, include, but are not limited to, type 1 insulin-dependent diabetes mellitus, inflammatory bowel disease, dermatitis, meningitis, thrombotic thrombocytopenic purpura, Sjögren's syndrome, encephalitis, uveitis, leukocyte adhesion deficiency, rheumatoid and other forms of immune arthritis, rheumatic fever, Reiter's syndrome, psoriatic arthritis, progressive systemic sclerosis, primary biliary cirrhosis, pemphigus, pemphigoid, necrotizing vasculitis, myasthenia gravis, multiple sclerosis, lupus erythematosus, polymyositis, sarcoidosis, granulomatosis, vasculitis, pernicious anemia, CNS inflammatory disorder, antigen-antibody complex mediated diseases, autoimmune hemolytic anemia, Hashimoto's thyroiditis, Graves disease, habitual spontaneous abortions, Reynard's syndrome, glomerulonephritis, dermatomyositis, chronic active hepatitis, celiac disease, autoimmune complications of AIDS, atrophic gastritis, ankylosing spondylitis and Addison's disease.

The method is also useful in treating non-malignant or immunological-related cell-proliferative diseases such as psoriasis, pemphigus vulgaris, Behcet's syndrome, acute respiratory distress syndrome (ARDS), ischemic heart disease, atherosclerosis, post-dialysis syndrome, leukemia, acquired immune deficiency syndrome, septic shock and other types of acute inflammation, and lipid histiocytosis. Essentially, any disorder which is etiologically linked to the pro-inflammatory process and cellular infiltration due to chemokine production (e.g., induction of IL-8, MIP-1α or β expression) would be considered susceptible to treatment.

The method of the invention is also useful for the treatment of microbial infections. Many microbes, such as bacteria, rickettsia, various parasites, and viruses, bind to vascular endothelium and leukocytes, and induce an inflammatory reaction resulting in production of interleukins for example. Thus, the type-2 CBP used in the method of the invention may be administered to a patient to prevent inflammation associated with such infections.

The dosage ranges for the administration of the type-2 CBP of the invention are those large enough to produce the desired effect in which the symptoms of the immune response show some degree of suppression. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counterindications. Dosage can vary from about 10 pg to 100 pg per dosage, in one or more dose administrations daily, for one or several days.

The type-2 CBP is administered by any suitable means, including parenteral, subcutaneous, intrapulmonary, intraareterial, intrarectal, intramuscular, and intranasal administration. Parenteral infusions include intramuscular, intravenous, intraarterial, or intraperitoneal administration. Type-2 CBP may also be administered transdermally in the form of a slow-release subcutaneous implant for example, or orally in the form of capsules, powders or granules. Type-2 CBP can also be administered by inhalation. For example, when used therapeutically for treatment of an inflammatory disorder of the lungs, a preferred route of administration would be by a pulmonary aerosol.

Pharmaceutically acceptable carrier preparations for parenteral administration include sterile or aqueous or non-aqueous solutions, suspensions, and emulsions. Examples of non-aqueous solvents are propylene glycol, polyethylene glycol, vegetable oils such as olive oil, and injectable organic esters such as ethyl oleate.

Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. The active therapeutic ingredient is often mixed with excipients which are pharmaceutically acceptable and compatible with the active ingredient. Suitable excipients include water, saline, dextrose, glycerol and ethanol, or combinations thereof. Intravenous vehicles include fluid and nutrient replenishers, electrolyte replenishers, such as those based on Ringer's dextrose, and the like. Preservatives and other additives may also be present such as, for example, antimicrobials, anti-oxidants, chelating agents, and inert gases and the like.

The invention also relates to a method for preparing a medicament or pharmaceutical composition comprising the type-2 CBP of the invention, the medicament being used for therapy of an undesirable immune response/inflammatory reaction wherein the immune response results in production of chemokines which bind to the type-2 CBP of the present invention.

The invention provides a pharmaceutical composition comprising at least one dose of an immunotherapeutically effective amount of an anti-inflammatory protein having a molecular weight of approximately 30-40 kD, depending on the extent of glycosylation, having amino acid sequence homology with the myxoma T1 interferon-y receptor homolog, and having the biological function of the myxoma T1 interferon-γ receptor homolog, in a pharmacological carrier.

The invention provides any pharmaceutical preparations and compositions containing the type-2 CBP of the invention for use in the method of the invention. The form will vary depending upon the route of administration. For example, compositions for injection can be provided in the form of an ampule, each containing a unit dose amount, or in the form of a container containing multiple doses.

Type-2 CBP can be formulated into the therapeutic composition as neutralized pharmaceutically acceptable salt forms. These include the acid addition salts which are formed with inorganic acids such as, for example, hydrochloric or phosphoric acid, or organic acids such as acetic, oxalic, tartaric and the like. Salts also include those formed from inorganic bases such as, for example, sodium, potassium, ammonium, calcium or ferric hydroxides, and organic bases such as isopropylamine, trimethylamine, histidine, procaine and the like.

Controlled delivery may be achieved by selecting appropriate macromolecules, for example, polyesters, polyamino acids, polyvinyl pyrrolidone, ethylenevinylacetate, methylcellulose, carboxymethylcellulose, protamine sulfate, or lactide/glycolide copolymers. The rate of release of the type-2 CBP may be controlled by altering the concentration of the macromolecule.

Another method for controlling the duration of action comprises incorporating the type-2 CBP into particles of a polymeric substance such as polyesters, polyamino acids, hydrogels, polylactide/glycolide copolymers, or ethylenevinylacetate copolymers. Alternatively, it is possible to entrap type-2 CBP in microcapsules prepared, for example, by coacervation techniques or by interfacial polymerization, for example, by the use of hydroxymethylcellulose or gelatin-microcapsules or poly(methylmethacrolate) microcapsules, respectively, or in a colloid drug delivery system. Colloidal dispersion systems include macromolecule complexes, nano-capsules, microspheres, beads, and lipid-based systems including oil-in-water emulsions, micelles, mixed micelles, and liposomes.

The following examples are intended to illustrate but not limit the invention. While they are typical of those that might be used, other procedures known to those skilled in the art may alternatively be used.

### EXAMPLE 1

### MATERIALS AND METHODS

### Rat model of injury induced atherosclerosis:

Sprague Dawley rats were induced to have balloon angioplasty mediated injury of the right or left iliofemoral artery. A 1.5mm USCI angioplasty balloon was advanced retrograde into the artery via cut down and arteriotomy under general pentobarbital anesthetic (6.5mg per 100g weight by i.m. injection, Somnotrol, MTC Pharmaceuticals, Cambridge , Ontario). 500pg of CBP (6 rats) or saline (6 rats) was given by intra-arterial injection of the CBP or control solution into the distal lumen of the angioplasty balloon catheter upstream from the site of subsequent balloon mediated damage. The balloon was then inflated to 8 bars pressure for 1.0 minutes. After angioplasty the balloon was deflated and withdrawn and the arteriotomy site closed with local application of n-butyl cyanoacrylate monomer (Nexaband, Veterinary Products Laboratories, Phoenix, Arizona). Each rat was maintained on a normal rat diet and was followed up for 4 weeks post surgery. At follow up, the rats were sacrificed with 2.0ml euthanyl per kg and the aorta was harvested for histological examination.

### Rabbit model of injury induced atherosclerosis:

Eight cholesterol fed New Zealand white rabbits are treated by balloon angioplasty of the distal abdominal aorta. All rabbits (strain New Zealand white) are fed 2% cholesterol in 10% peanut oil diet for 4 days/week, beginning 2 weeks before balloon injury. A 3-3.5mm angioplasty balloon catheter (≥ 1:1 ratio of balloon to aorta diameter) is introduced via femoral arterial cut down following anesthetic (40mg/kg ketalean, 8mg/kg xylazene, and 0.5mg/kg acepromazine by intramuscular injection). The balloon is inflated to 8 bars pressure in the distal abdominal aorta and advanced retrograde to the distal thoracic aorta. The balloon is advanced and withdrawn 3 times under fluoroscopic control in each rabbit to ensure endothelial denudation. Contrast angiograms are recorded prior to and after balloon angioplasty mediated trauma and at 4 weeks follow-up. Heparin (400 units) is given immediately after obtaining femoral access to decrease catheter associated thrombosis.

Purified type-2 CBP, 500pg per sample, is infused immediately after balloon mediated injury in the distal abdominal aorta of 4 rabbits. A parallel infusion of saline is infused locally into the distal abdominal aorta in 4 rabbits. Each infusate is administered via Wolinsky catheter in a total volume 10ml diluted in sterile 0.9% saline immediately following balloon mediated injury. All infusions are via a 3.25mm Wolinsky balloon (inflated to a final pressure of 6±1 bars for 2 minuets) in the abdominal aorta proximal to the iliac bifurcation. The Wolinsky balloon is positioned immediately above the iliac bifurcation under fluoroscopic control such that the perfusion balloon is routinely located from 0.5 - 2.5 cm above the bifurcation and designated as the primary infusion site. Upstream secondary sites are defined in the region above 2.5cm proximal to the iliac bifurcation. In all experiments, infusates are administered via Wolinsky catheter in a total volume of 10 ml diluted in sterile 0.9% saline immediately following balloon mediated injury. All infusions are via a 3.25mm Wolinsky balloon (inflated to a final pressure of 6±1 bars for 2 minutes) in the abdominal aorta proximal to the iliac bifurcation.

### Histology and morphometric analysis:

Histological analysis is performed at the primary site of Wolinsky infusion in the distal abdominal aorta (rabbits) or upper iliofemoral arterial branches (rat) representing the primary infusion site as defined by the original positioning of the perfusion balloon. In rabbits, internal control sections are taken from a downstream, non-infused site near the iliac bifurcation (0.5cm above the bifurcation to 0.5cm below the bifurcation) and in upstream, non-infused site (the upper abdominal aorta, 2.5cm-3.5cm above the iliac bifurcation). The area from 1.5-2.5cm above the iliac bifurcation is considered a border zone with potentially variable infusion doses due to balloon placement and is therefore not included in this analysis. In rats the primary balloon sites for both T-1 treated and saline infused rats were used for histological assessment. Hematoxylin and eosin staining of formalin fixed specimens was performed as previously described. Briefly, each specimen was fixed in 10% (v/v) sodium phosphate buffered formalin, processed, impregnated, embedded in paraffin and cut into 5µm sections by microtome. Sections from each specimen (a minimum of 2 sections per site) were then stained with hematoxylin and eosin and examined by light microscopy.

### EXAMPLE 2

### BINDING OF CYTOKINES TO A NOVEL VIRAL PROTEIN

Briefly, a variety of human cytokines were radiolabeled with ¹²⁵I, exposed to the secreted proteins harvested from control or poxvirus-infected BGMK cells, cross-linked, and then analyzed by SDS-PAGE for novel cytokine/protein complexes.

The cross-linking assay uncovered what was clearly a novel viral-specific protein that bound to each of the human chemokines that was tested: IL-8 and MIP-1β (FIGURE 2). FIGURE 2 (upper two panels) shows gel mobility shift assays using iodinated ligands and tissue culture supernatants. Tissue culture supernatants (Sups) were prepared as follows: BGMK (Baby Green Monkey Kidney Cells) left untreated (mock) or were infected with Myxoma (MYX), T7-deletion mutant of myxoma (myx-T7⁻ ), rabbit pox (RPV) or a 35kDa-deletion of RPV at a multiplicity of infection (MOI) of 3. Secreted proteins were prepared by washing the monolayer three times with PBS and replenishing it with serum free medium at 4h post infection; these supernatants were then collected at 18h post infection (L). Mock supernatants were prepared in the same way in the absence of virus. Supernatants were concentrated approximately 15 fold using Amicon concentrators. The human chemokines IL-8 and MIP-1β were labeled with ¹²⁵I using iodobeads (Pierce) according to the manufacturer's protocols.

The gel mobility shift assays were performed as follows: 5 µl of iodinated ligand was mixed with 10 µl SUP and allowed to sit at room temperature for 2 hours. then 2 µl of the chemical cross linking reagent 1-ethyl-3-(3-dimethylaminopropyl)-carbodiimide (EDC) (200mM in 100mM potassium phosphate, pH 7.5) was added for 15 minutes, followed by an additional 2µl for 15 minutes. The reaction was then quenched by the addition of 2µl of Tris-HCl (1.0M, pH 7.5). The resulting mixture was analyzed using SDS-PAGE and autoradiography. The arrows indicate the shifted complexes. The bottom panel indicates the Coomassie stained gels, showing the loss of the T7 protein in the myx-T7⁻ infection and the loss of the 35KDa protein in the RPV-35k⁻ infection.

### EXAMPLE 3

### ANALYSIS OF THE EFFICACY OF TYPE-2 CBP (T1) AS AN ANTI-RESTENOSIS PROTEIN AS SHOWN IN ANGIOPLASTY BALLOON MEDIATED INJURY IN RAT FEMORAL ARTERIES

Inflammation is associated with accelerated atherosclerotic plaque development in the arterial wall. There is a high rate of plaque recurrence, or restenosis, after the use of balloon angioplasty and other related angioplasty devices designed to open occluded arteries. Accelerated atherosclerotic plaque growth also has been reported under conditions leading to arterial injury, viral infections, vasculitis, homocystinuria, diabetes melitis, hypertension, hyperlipideuria, smoking and immune complex generated disorders. The larger DNA viruses have evolved mechanisms, such as anti-inflammatory proteins, that allow the virus to proliferate in the host with decreased inhibition by the host immune and inflammatory defense mechanisms. Type-2 CBP (T-1) was tested as a potential therapeutic agent for the prevention of plaque growth after angioplasty. Type-2 CBP is believed to act as both an interferon gamma receptor homologue and as a chemokine inhibitor. Type-2 CBP was tested in animal models of injury induced atherosclerosis and the results showed a significant decrease in plaque formation 4 weeks after infusion. The 35KDa protein, a Type-2 CBP isolated from Vaccinia virus, significantly decreased intimal hyperplasia (atherosclerotic plaque growth) after balloon angioplasty injury after a single intravenous injection (Table 1). This shows that type-2 CBP can be used as an anti-inflammatory agent for treatment of or prevention of immune based disorders.

10 Sprague Dawley rats were induced to have balloon injury of the right iliofemoral artery under general anesthesia. A 1.5mm angioplasty balloon was introduced via femoral arterial cutdown and the distal tip of the balloon was advanced to the iliac bifurcation. Immediately prior to inflating the balloon, 1.0ml of either saline (5 rats) or increasing concentrations of purified vaccinia 35K protein, at 50 pg (5 rats), was injected into the artery. The angioplasty balloon was then inflated to 6-8 atm for 2 minutes, deflated and removed. The femoral artery was sealed with nexaband at the puncture site after removal of the catheter. The rats were allowed to recover and were monitored for 4 weeks. At 4 weeks the rats were sacrificed and the arteries harvested for histological assessment. Intimal area was measured by morphometric analysis. A significant decrease in intimal hyperplasia (plaque area) was detected after 35KDa protein infusion in comparison with the control saline infusions (p≺0.0089) (Table 1).

**TABLE 1**

| Dose 35KDa | mean Plaque area mm² | S.E. | p value (t test) |
|---|---|---|---|
| 0 | 0.055 | 0.012 | NS |
| 50pg | 0.014 | 0.011 | 0.0089 |

### EXAMPLE 4

### CBP-II INHIBITS CHEMOKINE BINDING TO CELLULAR RECEPTORS OF HUMAN MONOCYTES

The inhibition of binding of human MIP-1α to surface receptor in primary human monocytes and THP-1 cells by 35KDa protein from vaccinia (strain Lister) (Table 2) and M-T1 (Table 3), respectively, was demonstrated according to the following protocol. (Note: experiment 3 in Tables 1 and 2 used THP-1 cells, all other results were obtained from primary monocytes.)

Radiolabeled ¹²⁵I MIP-1α (25µCi/ml) was obtained and an appropriate volume of radiolabeled (hot) MIP-1α was added such that each tube contains 50,000cpm. As a control, unlabeled (cold) MIP-1α was added (400ng) along with the hot MIP-1α to demonstrate background binding. To measure the inhibitory properties of 35KDa M-T1 (CBP-2) and M-T7 (CBP-1), varying doses of the inhibitor were added with the radiolabeled ligand and the samples were incubated at 37°C (5% CO₂) for 30 minutes. Primary monocytes isolated from human blood and separated on a Percoll gradient were diluted in RPMI 1640 containing 1% BSA to a concentration of 1x10⁷ cells/ml, and 200µl of these cells were added to each reaction. Similar concentrations were used for THP-1 cells (monocytic cell line). The sample tubes were rotated for 1 hour at room temperature, whereupon the cells were spun down at 13,000 rpm for 5 minutes. The supernatants were removed, and the cells washed with 800µl of 10% sucrose solution. The cells were again spun at 13,000 rpm for 10 minutes, and the supernatants removed. The radioactivity from the pellets was then measured using a gamma counter. As a second set of controls, the binding properties of H³-fMLP to its cellular receptor was also tested in the presence of the inhibitors (Table 4). The above protocol was followed, with the exception that a uniform volume of 1µl was distributed to each tube, which resulted in counts of approximately 180,000 cpm per tube. Note that 35KDa and M-T1 both quantitatively blocked binding of the chemokine to the cellular receptors of MIP-1α.

**TABLE 2**

| Surface Bound MIP-1α | | | | | | | | | | |
|---|---|---|---|---|---|---|---|---|---|---|
| Exp. | Molar excess of 35KDa protein | | | | | | | | Excess unlabeled MIP-1α | labeled MIP-1α |
| | 1600 | 800 | 400 | 200 | 50 | 40 | 4 | 2 | 6500X | |
| 1 | 80 | 77 | 107 | 144 | 1280 | --- | --- | --- | 470 | 4705 |
| 2 | --- | --- | --- | 88 | --- | 2342 | 10688 | 9113 | 410 | 7708 |
| 3 | 55 | --- | --- | --- | --- | --- | --- | --- | 1038 | 9944 |
| Note: Experiment 3 used THP-1 cells; Experiment 1 and 2 used primary monocytes. | | | | | | | | | | |

**TABLE 3**

| Surface Bound MIP-1α | | | | |
|---|---|---|---|---|
| Exp. | 200X Molar excess M-T1 | 200X Molar excess M-T7 | 6500X Molar excess unlabeled MIP-1α | Labeled MIP-1α |
| 1 | 978 | 10171 | 444 | 12206 |

**TABLE 4**

| Surface Bound fMLP | | | |
|---|---|---|---|
| Exp. | Labeled fMLP | 1x10⁶ M unlabeled fMLP | 35KDa (400ng) |
| 1 | 374 | 132 | 507 |

## Claims

1. A pharmaceutical composition comprising at least one dose of an immunotherapeutically effective amount of an anti-inflammatory protein having an amino acid sequence as set forth in SEQ ID NO:2 or a conservative variation of the polypeptide sequence in a pharmaceutically acceptable carrier.

2. The composition of claim 1, wherein the anti-inflammatory protein is a type 2 chemokine binding protein.

3. The composition of claim 2, wherein the chemokine is α class a or a class β chemokine.

4. The composition of claim 3, wherein the chemokine is selected from the group consisting of CTAP-III, gro/MGSA, ENA-78, MCP-1, interleukin-8, RANTES, MIP-1α, and MIP-1β, PF-4, IP-10, and NAP-2.

5. A method for identification of a chemokine, the method comprising contacting a chemokine binding protein having an amino acid sequence as set forth in SEQ ID NO:2 or a conservative variation of the polypeptide sequence with a composition suspected of being a chemokine and detecting binding of the chemokine composition to the chemokine binding protein.

6. The use of a therapeutically effective amount of a chemokine binding protein having an amino acid sequence as set forth in SEQ ID NO:2 or a conservative variation of the polypeptide sequence for the manufacture of a medicament for treating a chemokine-related immunopathological disorder selected from the group consisting of microbial infection, malignancy and metastasis, asthma, coronary restenosis, autoimmune diseases, cirrhosis, endotoxemia, atherosclerosis, reperfusion injury and inflammatory responses.

7. The use of claim 6, wherein the chemokine is a class α or a class β chemokine.

8. The use of claim 7, wherein the chemokine is selected from the group consisting of CTAP-III, gro/MGSA, ENA-78, MCP-1, interleukin-8, RANTES, MIP-1α, MIP-1β, PF-4, IP-10, and NAP-2.

9. The use of claim 6, wherein the pharmaceutical composition can be administered with an antibiotic or antiviral.

10. The use of claim 6, wherein the pharmaceutical composition can be administered subcutaneous, intravenous, intraarterial, intramuscular, intrarectal or transdermal.

## Patentansprüche

1. Eine pharmazeutische Zusammensetzung, umfassend mindestens eine Dosis einer immuntherapeutisch wirksamen Menge eines antiphlogistischen Proteins, das eine Aminosäuresequenz wie in SEQ ID NO:2 gezeigt oder eine konservative Variation der Polypeptidsequenz besitzt, in einem pharmazeutisch geeigneten Träger.

2. Zusammensetzung gemäß Anspruch 1, wobei das antiphlogistische Protein ein Typ 2 Chemokin-Bindungsprotein ist.

3. Zusammensetzung gemäß Anspruch 2, wobei das Chemokin ein Klasse α oder Klasse β Chemokin ist.

4. Zusammensetzung gemäß Anspruch 3, wobei das Chemokin ausgewählt ist aus der Gruppe bestehend aus CTAP-III, gro/MGSA, ENA-78, MCP-1, Interleukin-8, RANTES, MIP-1α, und MIP-1β, PF-4, IP-10, und NAP-2.

5. Verfahren zur Identifizierung eines Chemokins, umfassend in Kontakt bringen eines Chemokin-Bindungsproteins, das eine Aminosäuresequenz wie in SEQ ID NO:2 gezeigt oder eine konservative Variation der Polypeptidsequenz besitzt, mit einer Zusammensetzung, die ein Chemokin sein könnte und Nachweisen der Bindung der Chemokin-Zusammensetzung an das Chemokin-Bindungsprotein.

6. Verwendung einer therapeutisch wirksamen Menge eines Chemokin-Bindungsproteins, das eine Aminosäuresequenz wie in SEQ ID NO:2 gezeigt oder eine konservative Variation der Polypeptidsequenz besitzt, zur Herstellung eines Arzneimittels zur Behandlung einer Chemokin-bezogenen immunpathologischen Erkrankung ausgewählt aus der Gruppe bestehend aus mikrobieller Infektion, Malignität und Metastase, Asthma, koronarer Restenose, Autoimmunerkrankungen, Zirrhose, Endotoxämie, Atherosklerose, Reperfusionsschaden und Entzündungsreaktionen.

7. Verwendung gemäß Anspruch 6, wobei das Chemokin ein Klasse α oder Klasse β Chemokin ist.

8. Verwendung gemäß Anspruch 7, wobei das Chemokin ausgewählt ist aus der Gruppe bestehend aus CTAP-III, gro/MGSA, ENA-78, MCP-1, Interleukin-8, RANTES, MIP-1α, MIP-1β, PF-4, IP-10, und NAP-2.

9. Verwendung gemäß Anspruch 6, wobei die pharmazeutische Zusammensetzung mit einem Antibiotikum oder antiviralen Mittel verabreicht werden kann.

10. Verwendung gemäß Anspruch 6, wobei die pharmazeutische Zusammensetzung subkutan, intravenös, intraarteriell, intramuskulär, intrarektal oder transdermal verabreicht werden kann.

## Revendications

1. Composition pharmaceutique comprenant au moins une dose d'une quantité efficace du point de vue immunothérapeutique d'une protéine anti-inflammatoire ayant une séquence d'acides aminés telle qu'indiquée dans SEQ ID NO:2 ou une variation conservatrice de la séquence polypeptidique dans un support pharmaceutiquement acceptable.

2. Composition selon la revendication 1, dans laquelle la protéine anti-inflammatoire est une protéine de fixation de la chimiokine de type 2.

3. Composition selon la revendication 2, dans laquelle la chimiokine est une chimiokine de classe α ou une chimiokine de classe β.

4. Composition selon la revendication 3, dans laquelle la chimiokine est choisie dans le groupe consistant en CTAP-III, gro/MGSA, ENA-78, MCP-1, interleukine-8, RANTES, MIP-1α, et MIP-1β, PF-4, IP-10 et NAP-2.

5. Procédé pour l'identification d'une chimiokine, le procédé comprenant la mise en contact d'une protéine de fixation de chimiokine ayant une séquence d'acides aminés telle qu'indiquée dans SEQ ID NO:2 ou une variation conservatrice de la séquence polypeptidique avec une composition suspectée d'être une chimiokine et la détection de la fixation de la composition de chimiokine à la protéine de fixation de chimiokine.

6. Utilisation d'une quantité thérapeutiquement efficace d'une protéine de fixation de chimiokine ayant une séquence d'acides aminés telle qu'indiquée dans SEQ ID NO:2 ou une variation conservatrice de la séquence polypeptidique pour la production d'un médicament pour le traitement d'un trouble immunopathologique ayant un rapport avec la chimiokine, choisi dans le groupe consistant en une infection microbienne, une malignité et une métastase, l'asthme, la resténose coronarienne, les maladies auto-immunes, la cirrhose, l'endotoxémie, l'athérosclérose, un traumatisme de reperfusion et des réponses inflammatoires.

7. Utilisation selon la revendication 6, dans laquelle la chimiokine est une chimiokine de classe α ou une chimiokine de classe β.

8. Utilisation selon la revendication 7, dans laquelle la chimiokine est choisie dans le groupe consistant en CTAP-III, gro/MGSA, ENA-78, MCP-1, interleukine-8, RANTES, MIP-1α, MIP-1β, PF-4, IP-10 et NAP-2.

9. Utilisation selon la revendication 6, dans laquelle la composition pharmaceutique peut être administrée avec un antibiotique ou un agent antiviral.

10. Utilisation selon la revendication 6, dans laquelle la composition pharmaceutique peut être administrée par voie sous-cutanée, intraveineuse, intra-artérielle, intramusculaire, intrarectale ou transdermique.
